# EUROPEAN PATENT SPECIFICATION

(11) **EP 2 683 360 B1**
(45) Date of publication and mention of the grant of the patent: **24.02.2016**
(21) Application number: 12757055.4
(22) Date of filing: 08.03.2012
(51) Int. Cl.: A61K 47/48, A61K 9/00, A61K 38/17, A61P 9/00

(54) **PEGYLATED APELIN AND USES THEREOF**
PEGYLIERTES APELIN UND VERWENDUNGEN DAVON
APELINE PÉGYLÉE ET SES UTILISATIONS

(30) Priority: 11.03.2011 US 201161451623 P
(43) Date of publication of application: 15.01.2014
(73) Proprietor: Genzyme Corporation, Cambridge, MA 02142 (US)
(72) Inventor: JIA, Zhiqiang, Cambridge, Massachusetts 02142 (US); HOU, Lihui, Cambridge, Massachusetts 02142 (US); PAN, Clark Q., Cambridge, Massachusetts 02142 (US); AKITA, Geoffrey Y., Cambridge, Massachusetts 02142 (US)
(74) Representative: Pearl Cohen Zedek Latzer Baratz UK LLP
(86) International application number: PCT/US2012/028298
(87) International publication number: WO 2012/125408

(56) References cited:
- US-A1- 2004 219 152
- US-A1- 2008 182 779
- US-A1- 2009 252 703
- US-A1- 2009 286 723
- US-B1- 6 420 339
- Z.Q. JIA ET AL: "Cardiovascular effects of a PEGylated apelin", PEPTIDES, vol. 38, no. 1, 1 November 2012 (2012-11-01), pages 181-188, XP055131938, ISSN: 0196-9781, DOI: 10.1016/j.peptides.2012.09.003
- I. SZOKODI: "Apelin, the Novel Endogenous Ligand of the Orphan Receptor APJ, Regulates Cardiac Contractility", CIRCULATION RESEARCH, vol. 91, no. 5, 15 August 2002 (2002-08-15), pages 434-440, XP55132065, ISSN: 0009-7330, DOI: 10.1161/01.RES.0000033522.37861.69
- M. F. BERRY: "Apelin Has In Vivo Inotropic Effects on Normal and Failing Hearts", CIRCULATION, vol. 110, no. 11_suppl_1, 14 September 2004 (2004-09-14), pages II-187, XP55132066, ISSN: 0009-7322, DOI: 10.1161/01.CIR.0000138382.57325.5c
- ROBERTS M J ET AL: "Chemistry for peptide and protein PEGylation", ADVANCED DRUG DELIVERY REVIEWS, ELSEVIER, AMSTERDAM, NL, vol. 54, no. 4, 17 June 2002 (2002-06-17), pages 459-476, XP002293146, ISSN: 0169-409X, DOI: 10.1016/S0169-409X(02)00022-4
- DATABASE GENBANK [Online] 15 August 2001 LEE ET AL.: 'Homo sapiens apelin gene, complete cds.', XP003030210 Database accession no. AF179680.
- CANALLE ET AL.: 'Polypeptide-polymer bioconjugates.' CHEM. SOC. REV vol. 39, no. 1, 2010, pages 329 - 353, XP055122667

## Description

### CROSS-REFERENCE TO RELATED APPLICATIONS

This application claims priority to United States Provisional Patent Application 61/451,623, filed March 11, 2011.

### FIELD OF THE INVENTION

The invention relates to compositions for treating a disease or disorder associated with Apelin. Specifically, the invention relates to a pegylated form of Apelin to provide extended circulating life and inotropic effects, and thereby efficiently treat diseases or disorders associated with Apelin.

### BACKGROUND OF THE INVENTION

Apelin, a peptide initially isolated from bovine stomach extracts, acts as an endogenous ligand for G protein coupled APJ receptor. Apelin gene encodes a pre-proprotein of 77 amino acids, with a signal peptide in the N-terminal region. After translocation into the endoplasmic reticulum and cleavage of the signal peptide, the proprotein of 55 amino acids may generate several active fragments: a 36 amino acid peptide corresponding to the sequence 42-77 (Apelin 36), a 17 amino acid peptide corresponding to the sequence 61-77 (Apelin 17) and a 13 amino acid peptide corresponding to the sequence 65-77 (Apelin 13).

Apelin and its receptor are expressed in majority of tissues and organs, including cardiovascular system. Multiple effects relevant to cardiovascular system have been reported, including positive inotropic activities, diuretic effect, and direct myocardial protection from ischemia reperfusion injury. As for its effect on blood pressure, the reports are conflicting. Some of the studies showed decreased arterial pressure via a NO-dependent mechanism, but there are also contradictory result reported with Apelin 13 increasing the arterial pressure, as well as a biphasic change of mean arterial blood pressure.

Several studies have documented cardiovascular effects of Apelin, including enhanced inotropy and vasodilation. However, these cardiovascular effects are short lived due to the short circulating life of the Apelin peptide.

Accordingly, there exists a need for improved Apelin compositions so as to improve their circulating life, inotropic effects, and other beneficial characteristics.

### SUMMARY OF THE INVENTION

The invention provides a pegylated Apelin 36 that comprises one or more polyethylene glycol (PEG) molecules operably linked to at least one amino acid residue in the N-terminal of the Apelin 36. The pegylated Apelin has a prolonged circulating life and inotropic effect, relative to a non-pegylated Apelin.

Additionally, the invention provides methods for producing the pegylated Apelin by reacting an Apelin 36 with an activated PEG-aldehyde linker in the presence of a reducing agent to form the pegylated Apelin 36 under conditions in which the linker is covalently attached to at least one amino acid residue in the N-terminal of said Apelin.

The invention also provides pharmaceutical compositions that comprise a therapeutically effective amount of the pegylated Apelin 36.

The invention further provides kits that comprise a therapeutically effective amount of the pegylated Apelin.

The invention further provides a therapeutically effective amount of the pegylated Apelin 36 for use in treating a disease or disorder associated with an Apelin, in a subject, wherein the disease or disorder is a cardiovascular disease, an ischemia-reperfusion injury, myocardial infarction, acute decompensated heart failure, chronic heart failure, cardiomyopathy, endocrine disorder, metabolic disorder or pulmonary hypertension.

Other features and advantages of the present invention will become apparent from the following detailed description examples and figures. It should be understood, however, that the detailed description and the specific examples while indicating preferred embodiments of the invention are given by way of illustration only.

### BRIEF DESCRIPTION OF THE DRAWINGS

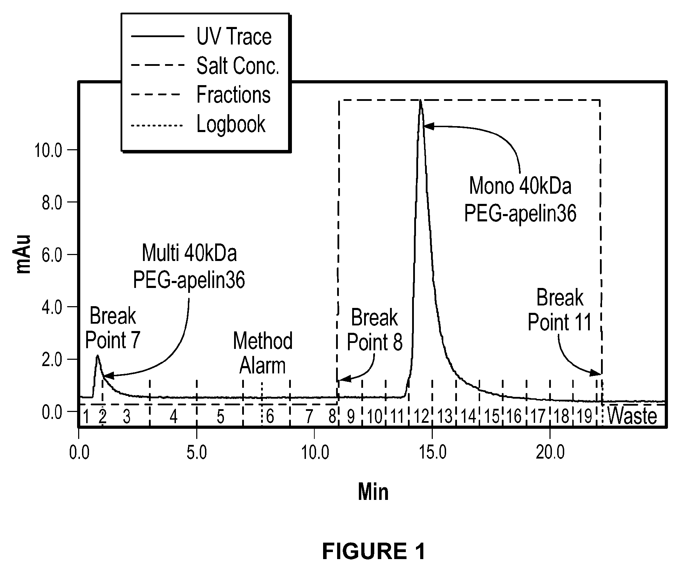

Figure 1. The column profile of Weak Cation Exchanger chromatography of the 40kDa PEG-apelin-36.

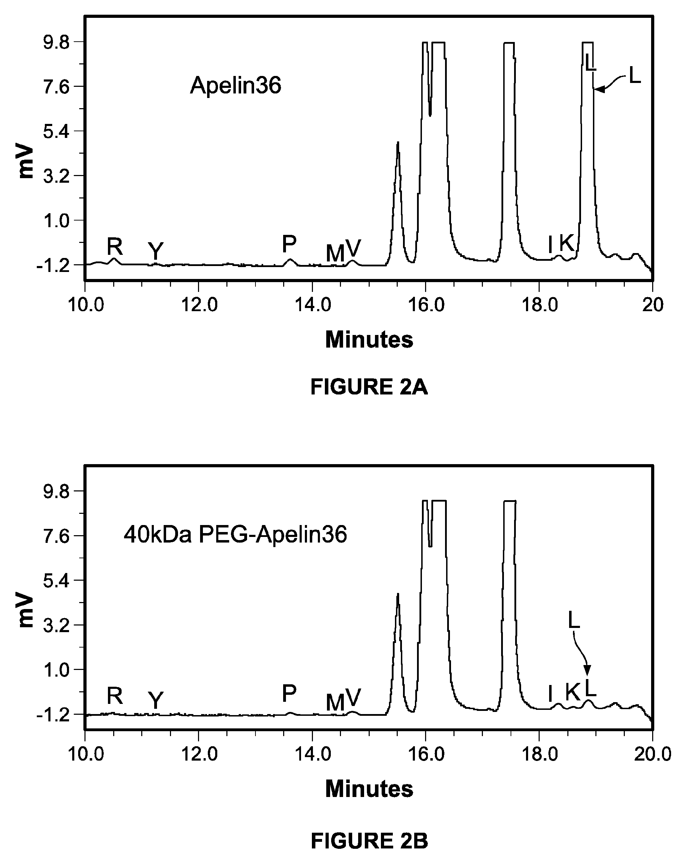

Figure 2. N-terminal sequencing showed selective PEGylation of apelin-36 at the N-terminus. For apelin-36, the first residue L, was detected in the first cycle (the spike indicated by solid arrow in the upper panel). However, only 2% recovery of first residue L was obtained from 40kDa PEG-apelin-36 conjugate (arrow in the lower panel).

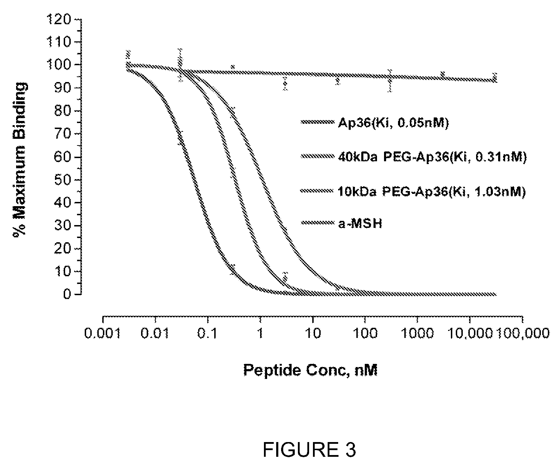

Figure 3. Competitive radioligand binding to APJ receptor using apelin, its PEG conjugates, and α-MSH, a negative control.

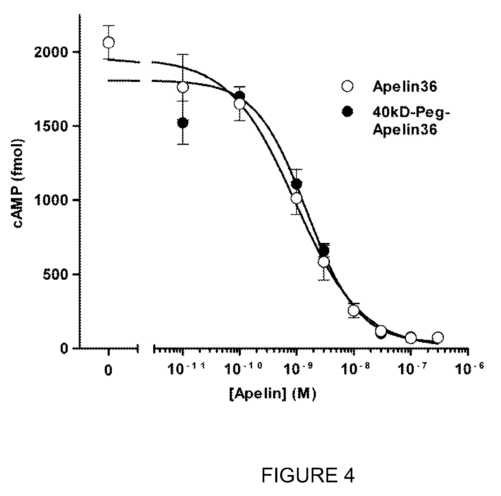

Figure 4. Attachment of an N-terminal PEG group to apelin-36 does not compromise its function as an APJ agonist. In a forskolin-mediated cAMP accumulation assay the IC50 of PEG-apelin-36 was shifted 1.5-fold to the right, with no change in the maximal inhibition of forskolin-mediated cAMP accumulation versus unmodified apelin-36.

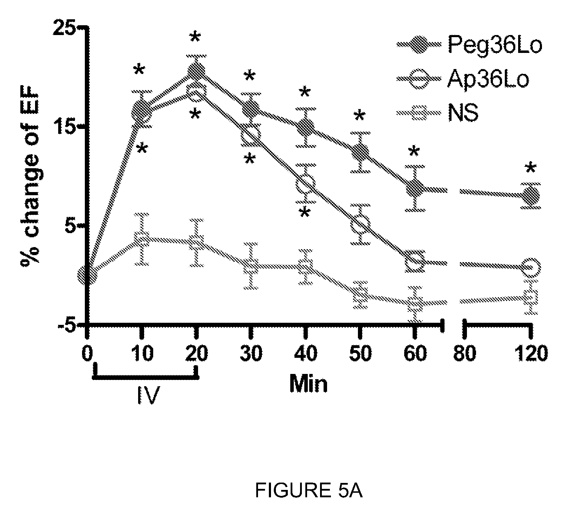

Figure 5a. PEG-apelin36 resulted in prolonged inotropic effects in rats. In normal rats that received 30 nM PEG-apelin-36 (Peg36Lo) EF was increased compared to baseline (BL) and normal saline (NS) at all time points. In animals that received 30 nM apelin-36 (Ap36Lo) EF was not significantly different from BL and NS at 50 min. * p<0.05 vs BL and time matched NS; IV= intravenous infusion.

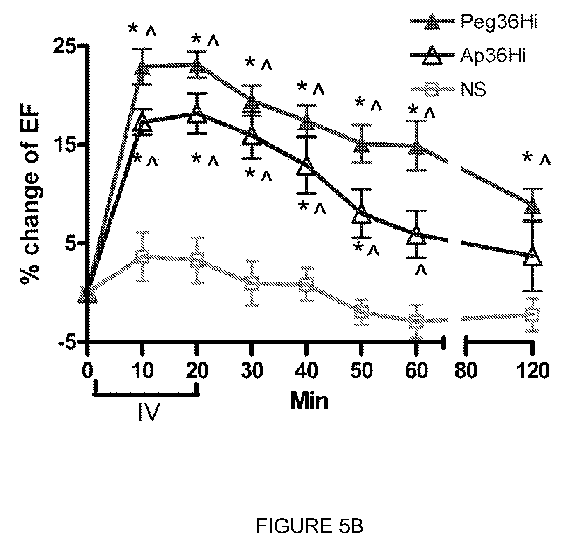

Figure 5b. In normal rats that received 300 nM PEG-apelin-36 (Peg36Hi) EF was increased compared to the baseline (BL) and normal saline (NS) at all time points. In animals that received 300nM apelin-36 (Ap36Hi) EF was not significantly different from BL post at 50 min, and was not significantly from NS post 60 min. * P<0.05 vs BL; ^ P<0.05 vs time matched NS; IV = intravenous infusion.

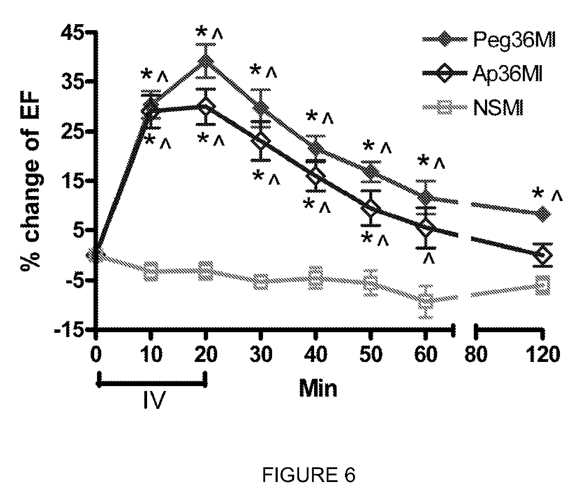

Figure 6. PEG-apelin-36 resulted in prolonged inotropic effects in Myocardial Infarct (MI) rats. In MI rats that received PEG-apelin-36 (Peg36MI) EF was increased compared to baseline (BL) and normal saline (NSMI) at all time points. In MI rats that received apelin 36 (Ap36MI) EF was not significantly different from BL post 50 min and was not significantly different from the NSMI post 60 min. * P<0.05 vs BL; ^ P<0.05 vs time matched NSMI; IV=intravenous infusion.

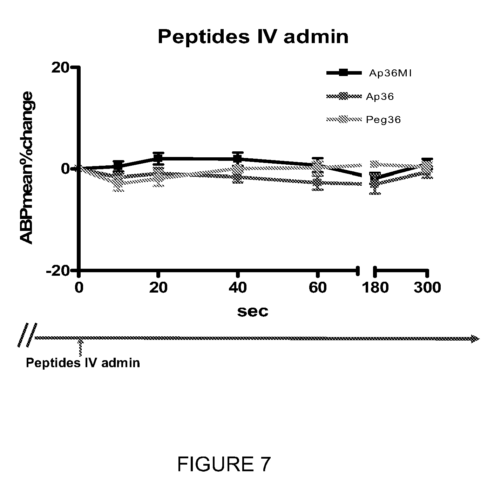

Figure 7. IV injection of PEG-apelin-36 (Peg36) or apelin-36 (Ap36) in normal rats; and apelin 36 in MI rats (Ap36MI) did not result in changes in mean aortic blood pressure ( ABPmean).

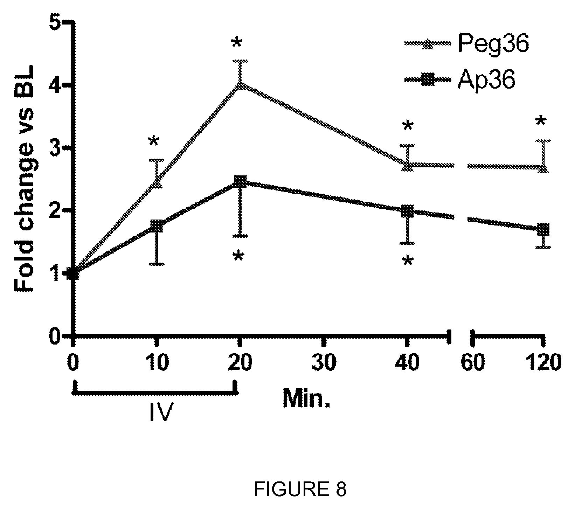

Figure 8. PEG-apelin-36 (Peg36) has a longer circulating life than apelin-36 (Ap36). In the Peg36 group, the blood apelin-36 was increased compared to baseline at all time points. * p<0.05 vs baseline.

### DETAILED DESCRIPTION OF THE INVENTION

The invention relates to compositions for treating a disease or disorder associated with Apelin. Specifically, the invention relates to a pegylated form of Apelin 36 to provide extended circulating life and inotropic effects, and thereby efficiently treat diseases or disorders associated with Apelin.

In one embodiment, provided herein is a pegylated Apelin 36 molecule comprising one or more polyethylene glycol (PEG) molecules operably linked to at least one amino acid residue in the N-terminal of the Apelin 36.

In another embodiment, provided herein is a method for producing a pegylated Apelin 36, comprising the step of: reacting an Apelin 36 with an activated PEG-aldehyde linker in the presence of a reducing agent to form said pegylated Apelin36 under conditions in which the linker is covalently attached to at least one amino acid residue in the N-terminal of said Apelin 36.

In another embodiment, provided herein is a pharmaceutical composition comprising a therapeutically effective amount of a pegylated Apelin 36 that comprises one or more polyethylene glycol (PEG) molecules operably linked to at least one amino acid residue in the N-terminal of the Apelin 36.

In another embodiment, provided herein is a therapeutically effective amount of a pegylated Apelin 36 that comprises one or more polyethylene glycol (PEG) molecules operably linked to at least one amino acid residue in the N-terminal of said Apelin 36 for use in treating a disease or disorder associated with an Apelin in a subject, wherein the disease or disorder is a cardiovascular disease, an ischemia-reperfusion injury, myocardial infarction, acute decompensated heart failure, chronic heart failure, cardiomyopathy, endocrine disorder, metabolic disorder or pulmonary hypertension.

To address the limitations in Apelin peptide characteristics, a 40 KDa PEGylated Apelin was successfully produced, characterized, and evaluated *in vitro* and *in vivo.* The inventors of the instant application surprisingly and unexpectedly found that compared to Apelin, PEGylated Apelin exhibits an extended circulating life and correspondingly, an extended inotropic effect following IV administration.

Apelin is a well known protein. The amino acid and nucleic acid sequences of Apelin are well known in the art. For example, GenBank Identification Numbers AAF25815.1 and AF179680 contain Apelin amino acid and nucleic acid sequences, respectively.

In one embodiment, Apelin comprises the amino acid sequence set forth in SEQ ID NO: 1.

In another embodiment, Apelin is encoded by the nucleic acid sequence set forth in SEQ ID NO: 2.

The term Apelin 36, as used herein, refers to the 36 amino acid peptide of amino acid residues 42-77 of SEQ ID NO: 1.

The one or more PEG molecules may be operably linked to an Apelin peptide through any known linking method. For example, two or more PEG molecules may be operably linked through a simple covalent bond, a flexible peptide linker or a disulfide bridge. Peptide linkers may be entirely artificial (e.g., comprising 2 to 20 amino acid residues independently selected from the group consisting of glycine, serine, asparagine, threonine and alanine) or adopted from naturally occurring proteins.

In one embodiment, an Apelin peptide is reacted with an activated PEG-aldehyde linker in the presence of a reducing agent (e.g., cyanoborohydride) to form a pegylated Apelin under conditions in which the linker is covalently attached to an amino acid residue (e.g., Leucine) in the N-terminal of Apelin.

PEGylation of the molecules can be carried out, e.g., according to the methods described in Youngster et al., Curr Pharm Des (2002), 8:2139; Grace et al., J Interferon Cytokine Res (2001), 21:1103; Pepinsky et al., J Pharmacol Exp Ther (2001), 297:1059; Pettit et al., J Biol Chem (1997), 272:2312; Goodson et al. Biotechnology NY (1990), 8:343; Katre; J Immunol (1990), 144:209).

Any kind of polyethylene glycol is suitable for the present invention provided that the PEG-polypeptide is still functionally active which can be assayed according to methods known in the art. Preferably, the polyethylene glycol of the present invention is PEG 1000, 2000, 3000, 5000, 10000, 15000, 20000, 30000 or 40000 with PEG 30000 or 40000 being particularly preferred.

In one example, the pegylated molecule comprises a monomeric Apelin. In another example, the pegylated molecule comprises is an oligomeric Apelin. In yet another example, the pegylated molecule comprises a multiarm PEG, wherein one or more monomeric Apelin are operably linked to the multiarm PEG.

The Apelin of the present invention according to one embodiment of the invention is recombinantly produced by use of a polynucleotide encoding the Apelin and vectors, preferably expression vectors containing said polynucleotides. For the production of the oligomers of the invention, the polynucleotides are obtained from existing clones, i.e., preferably encode the naturally occurring polypeptide or a part thereof. Polypeptides encoded by any polynucleotide which hybridises to the complement of the native DNA or RNA under highly stringent or moderate stringent conditions (for definitions, see Sambrook, Molecular Cloning A Laboratory Manual, Cold Spring Harbor Laboratory (1989) N.Y.) as long as that polypeptide maintains the biological activity of the native sequence, are also useful for producing the oligomers of the present invention.

The Apelin peptides can also be prepared by solid phase synthesis according to methods well known to a person skilled in the art, see, e.g., Coligan (ed) et al., Current Protocols in Protein Science, John Wiley and Sons, Inc., (2001) N.J. For example, the peptides apelin-13 and apelin-36 are prepared by solid phase synthesis using an Fmoc strategy on a 430A peptide synthesizer (Applied Biosystems, Foster City, CA) and a 9050 Pepsynthesizer Plus (Perseptive Biosystems, Cambridge, MA). Crude peptides are purified by preparative reverse phase high-performance liquid chromatography. Fractions containing the appropriate peptide are pooled and lyophilized. The purity of the final product is assessed by analytical reverse phase high-performance liquid chromatography, capillary electrophoresis, and matrix-assisted laser desorption ionization time-of-flight mass spectrometry.

The recombinant vectors can be constructed according to methods well known to the person skilled in the art; see, e.g., Sambrook, Molecular Cloning A Laboratory Manual, Cold Spring Harbor Laboratory (1989) N.Y. A variety of expression vector/host systems may be utilized to contain and express sequences encoding the oligomers of the present invention.

Exemplary vectors include plasmids, phagemids, cosmids, viruses and phage nucleic acids or other nucleic acid molecules that are capable of replication in a prokaryotic or eukaryotic host. The vectors typically contain a marker to provide a phenotypic trait for selection of transformed hosts such as conferring resistance to antibiotics such as ampicillin or neomycin

The vector may be an expression vector, wherein the nucleic acid encoding the peptide is operably linked to an expression control sequence. Typical expression vectors contain transcription and translation terminators, initiation sequences, and promoters useful for regulation of the expression of the nucleic acid molecules of the invention. The vectors may also contain genetic expression cassettes containing an independent terminator sequence, sequences permitting replication of the vector in both eukaryotes and prokaryotes, *i.e.*, shuttle vectors and selection markers for both prokaryotic and eukaryotic systems.

Suitable promoters include constitutive promoters and inducible promoters. Representative expression control sequences/promoters include the *lac* system, the *trp* system, the *tac* system, the *trc* system, major operator and promoter regions of phage lambda, the control region of *fd* coat protein, the glycolytic promoters of yeast, *e.g.*, the promoter for 3-phosphoglycerate kinase, the promoters of yeast acid phosphatase, *e.g.*, *Pho5*, the promoters of the yeast alpha mating factors, promoters derived from the human cytomegalovirus, metallothionine promoter, murine mammary tumor virus promoter, Rous sarcoma virus promoter, polyhedrin promoter and promoters derived from polyoma, adenovirus, retrovirus, and simian virus, *e.g.*, the early and late promoters of SV40.

The invention also includes non-human hosts such as cells or organisms containing a nucleic acid molecule or a vector of the invention. By "host" it is meant a non-human unicellular or multicellular organism or a "host cell", which refers to a cell or population of cells into which a nucleic acid molecule or vector of the invention is introduced. "A population of host cells" refers to a group of cultured cells into which a nucleic acid molecule or vector of the present invention can be introduced and expressed.

A host of the present invention may be prokaryotic or eukaryotic. Suitable prokaryotic hosts include, for example, *E. coli,* such as *E. coli* SG-936, *E. coli* HB 101, *E. coli* W3110, *E. coli* X1776, *E. coli* X2282, *E. coli* DHI, and *E. coli* MRC1, *Pseudomonas*, *Bacillus,* such as *Bacillus subtilis*, and *Streptomyces*. Suitable eukaryotic cells include yeast and other fungi, insect cells, plant cells, human cells, and animal cells, including mammalian cells, such as hybridoma lines, COS cells, NS0 cells and CHO cells.

The invention also includes methods of producing an Apelin, the method comprising: culturing a host cell; and recovering the Apelin from said host cell.

Depending on the expression system and host selected, the molecules are produced by growing host cells transformed by an expression vector described above whereby the protein is expressed. The expressed protein is then isolated from the host cells and purified. If the expression system secretes the protein into growth media, the product can be purified directly from the media. If it is not secreted, it can be isolated from cell lysates. The selection of the appropriate growth conditions and recovery methods are within the skill of the art. For example, once expressed, the product may be isolated and purified by any number of techniques, well known in the art. A protein (e.g., Apelin) of the present invention obtained as above may be isolated from the interior or exterior (e.g., medium) of the cells or hosts, and purified as a substantially pure homogeneous protein. The method for protein isolation and purification is not limited to any specific method. In fact, any standard method may be used. For instance, column chromatography, filtration, ultrafiltration, salt precipitation, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the protein.

For chromatography, for example, affinity chromatography, ion-exchange chromatography, hydrophobic chromatography, gel filtration, reverse phase chromatography, adsorption chromatography, and such may be used (ed. Daniel R. Marshak et al. (1996) Strategies for Protein Purification and Characterization: A Laboratory Course Manual., Cold Spring Harbor Laboratory Press). These chromatographies may be performed by liquid chromatography, such as, HPLC and FPLC. Thus, the present invention provides highly purified proteins produced by the above methods.

The invention also provides a pharmaceutical composition comprising the oligomer, nucleic acid, vector, or host cell of this invention and one or more pharmaceutically acceptable carriers. "Pharmaceutically acceptable carriers" include any excipient which is nontoxic to the cell or mammal being exposed thereto at the dosages and concentrations employed. The pharmaceutical composition may include one or additional therapeutic agents.

Pharmaceutically acceptable carriers include solvents, dispersion media, buffers, coatings, antibacterial and antifungal agents, wetting agents, preservatives, buggers, chelating agents, antioxidants, isotonic agents and absorption delaying agents.

Pharmaceutically acceptable carriers include water; saline; phosphate buffered saline; dextrose; glycerol; alcohols such as ethanol and isopropanol; phosphate, citrate and other organic acids; ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; EDTA; salt forming counterions such as sodium; and/or nonionic surfactants such as TWEEN, polyethylene glycol (PEG), and PLURONICS; isotonic agents such as sugars, polyalcohols such as mannitol and sorbitol, and sodium chloride; as well as combinations thereof. Antibacterial and antifungal agents include parabens, chlorobutanol, phenol, ascorbic acid, and thimerosal.

The pharmaceutical compositions of the invention may be formulated in a variety of ways, including for example, liquid, semi-solid and solid dosage forms, such as liquid solutions (e.g., injectable and infusible solutions), dispersions or suspensions, tablets, pills, powders, liposomes and suppositories. In some embodiments, the compositions are in the form of injectable or infusible solutions. The composition is in a form suitable for oral, intravenous, intraarterial, intramuscular, subcutaneous, parenteral, transmucosal, transdermal, or topical administration. The composition may be formulated as an immediate, controlled, extended or delayed release composition.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. In the subject invention, pharmaceutically acceptable carriers include, but are not limited to, 0.01-0.1M and preferably 0.05M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present such as for example, antimicrobials, antioxidants, chelating agents, and inert gases and the like.

More particularly, pharmaceutical compositions suitable for injectable use include sterile aqueous solutions (where water soluble) or dispersions and sterile powders for the extemporaneous preparation of sterile injectable solutions or dispersions. In such cases, the composition must be sterile and should be fluid to the extent that easy syringability exists. It should be stable under the conditions of manufacture and storage and will preferably be preserved against the contaminating action of microorganisms, such as bacteria and fungi. The carrier can be a solvent or dispersion medium containing, for example, water, ethanol, polyol (*e.g.*, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Suitable formulations for use in the therapeutic methods disclosed herein are described in Remington's Pharmaceutical Sciences, Mack Publishing Co., 16th ed. (1980).

In some embodiments, the composition includes isotonic agents, for example, sugars, polyalcohols, such as mannitol, sorbitol, or sodium chloride. Prolonged absorption of the injectable compositions can be brought about by including in the composition an agent which delays absorption, for example, aluminum monostearate and gelatin.

Sterile injectable solutions can be prepared by incorporating the molecule, by itself or in combination with other active agents, in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, one method of preparation is vacuum drying and freeze-drying, which yields a powder of an active ingredient plus any additional desired ingredient from a previously sterile-filtered solution thereof. The preparations for injections are processed, filled into containers such as ampoules, bags, bottles, syringes or vials, and sealed under aseptic conditions according to methods known in the art. Further, the preparations may be packaged and sold in the form of a kit such as those described in US Appl. Publ. No. 2002/0102208 A1. Such articles of manufacture will preferably have labels or package inserts indicating that the associated compositions are useful for treating a subject suffering from, or predisposed to autoimmune or neoplastic disorders.

Effective doses of the compositions of the present invention, for treatment of conditions or diseases as described herein vary depending upon many different factors, including means of administration, target site, physiological state of the patient, whether the patient is human or an animal, other medications administered, and whether treatment is prophylactic or therapeutic. Usually, the patient is a human but non-human mammals including transgenic mammals can also be treated. Treatment dosages may be titrated using routine methods known to those of skill in the art to optimize safety and efficacy.

The pharmaceutical compositions of the invention may include a "therapeutically effective amount." A "therapeutically effective amount" refers to an amount effective, at dosages and for periods of time necessary, to achieve the desired therapeutic result. A therapeutically effective amount of a molecule may vary according to factors such as the disease state, age, sex, and weight of the individual, and the ability of the molecule to elicit a desired response in the individual. A therapeutically effective amount is also one in which any toxic or detrimental effects of the molecule are outweighed by the therapeutically beneficial effects.

The invention further provides a therapeutically effective amount of the pegylated Apelin 36 molecule for use in treating a disease or condition associated with an Apelin, wherein the disease or condition is a cardiovascular disease, an ischemia-reperfusion injury, a myocardial infarction, an acute decompensated heart failure, a chronic heart failure, a cardiomyopathy, an endocrine/metabolic disorder or pulmonary hypertension.

As used herein, the terms "treat" and "treatment" refer to therapeutic treatment, including prophylactic or preventative measures, wherein the object is to prevent or slow down (lessen) an undesired physiological change associated with a disease or condition. Beneficial or desired clinical results include, but are not limited to, alleviation of symptoms, diminishment of the extent of a disease or condition, stabilization of a disease or condition (*i.e.*, where the disease or condition does not worsen), delay or slowing of the progression of a disease or condition, amelioration or palliation of the disease or condition, and remission (whether partial or total) of the disease or condition, whether detectable or undetectable. "Treatment" can also mean prolonging survival as compared to expected survival if not receiving treatment. Those in need of treatment include those already with the disease or condition as well as those prone to having the disease or condition or those in which the disease or condition is to be prevented.

The pegylated Apelin may be administered alone, or in combination with one or more therapeutically effective agents or treatments. The other therapeutically effective agent may be conjugated to the pegylated Apelin, incorporated into the same composition as the pegylated Apelin, or may be administered as a separate composition. The other therapeutically agent or treatment may be administered prior to, during and/or after the administration of the pegylated Apelin.

The administration of the pegylated Apelin with other agents and/or treatments may occur simultaneously, or separately, via the same or different route, at the same or different times. Dosage regimens may be adjusted to provide the optimum desired response (*e.g.*, a therapeutic or prophylactic response).

In one example, a single bolus or infusion may be administered. In another example, several divided doses may be administered over time. In yet another example, a dose may be proportionally reduced or increased as indicated by the exigencies of the therapeutic situation. Dosage unit form, as used herein, refers to physically discrete units suited as unitary dosages for treating mammalian subjects. Each unit may contain a predetermined quantity of active compound calculated to produce a desired therapeutic effect. In some embodiments, the dosage unit forms of the invention are dictated by and directly dependent on the unique characteristics of the active compound and the particular therapeutic or prophylactic effect to be achieved.

The composition of the invention may be administered only once, or it may be administered multiple times. For multiple dosages, the composition may be, for example, administered three times a day, twice a day, once a day, once every two days, twice a week, weekly, once every two weeks, or monthly.

It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

"Administration" to a subject is not limited to any particular delivery system and may include, without limitation, parenteral (including subcutaneous, intravenous, intramedullary, intraarticular, intramuscular, or intraperitoneal injection) rectal, topical, transdermal or oral (for example, in capsules, suspensions or tablets). Administration to a host may occur in a single dose or in repeat administrations, and in any of a variety of physiologically acceptable salt forms, and/or with an acceptable pharmaceutical carrier and/or additive as part of a pharmaceutical composition (described earlier). Once again, physiologically acceptable salt forms and standard pharmaceutical formulation techniques are well known to persons skilled in the art (see, for example, Remington's Pharmaceutical Sciences, Mack Publishing Co.).

The composition of the invention (i.e., pegylated Apelin 36) may be administered parenterally (e.g., intravenous, subcutaneous, intraperitoneal, intramuscular). Further, the composition of the invention may be administered by intravenous infusion or injection. The composition of the invention may be administered by intramuscular or subcutaneous injection. In some embodiments, the composition of the invention may be administered orally. As used herein, a "composition" refers to any composition that contains a pharmaceutically effective amount of a pegylated Apelin.

The treatment described herein can be used to treat any suitable mammal, including primates, such as monkeys and humans, horses, cows, cats, dogs, rabbits, and rodents such as rats and mice. In one embodiment, the mammal to be treated is human.

The following examples are presented in order to more fully illustrate the preferred embodiments of the invention.

### EXAMPLES

### EXAMPLE 1

### PEG Conjugated Apelin 36

A 40 kDa PEG conjugated apelin-36 (PEG-apelin-36) was successfully produced with N-terminal conjugation, high purity (>98%) and minimum reduction of APJ receptor binding affinity Using an adenylate cyclase inhibition assay, comparable in vitro bioactivity was observed between the PEG-apelin-36 and unmodified apelin-36. In vivo evaluation of the PEG-apelin-36 was performed in normal rats and rats with myocardial infarction (MI). Cardiac function was assessed via echocardiography before, during a 20 minutes IV infusion and up to 100 minutes post peptide infusion. Similar increases in cardiac ejection fraction (EF) were observed during the infusion of PEG-apelin-36 and apelin-36 in normal rats. However, animals that received PEG-apelin-36 maintained significantly increased EF over the 100 minute post infusion monitoring period compared to the animals that received unmodified apelin-36. Interestingly, EF increases observed with PEG-apelin-36 and apelin-36 were greater in the MI rats. PEG-apelin-36 had a prolonged circulating life compared to apelin-36 in rats. There were no changes in aortic blood pressure when PEG-apelin-36 or apelin-36 was administered.

### EXAMPLE 2

### Materials and methods:

### PEGylation of apelin

*Conjugation of apelin-36 (Ana Spec, Fremont*, *CA)*: The conjugation with 10kDa, 30kDa and 40kDa branched aldehyde PEGs (NOF America Corp., White Plains, NY) were carried out at 0.2uM peptide and 0.4 uM PEG in the presence of 20mM cyanoborohydride (Sigma-Aldrich, St. Louis, MO) in 0.1 M sodium acetate buffer at pH 5. The conjugation was conducted at 25°C overnight and quenched with 2mM Tris at pH 7.5. The conjugates were dialyzed against water to remove the unconjugated peptide. The 40kDa PEGylated apelin-36 conjugate (PEG-apelin-36) was further purified by weak cation exchange chromatography on a HiTrap SP FF column (G.E.Healthcare, Piscataway, NJ) to remove free PEG. Conjugate was eluted at 1.5M salt concentration from 15 minute column run. Nanodrop A280 was used to determine the concentration of the apelin-36 conjugate.

*Determination of the site of PEGylation:* 200 pmoles of apelin-36 and PEG-apelin-36 were N-terminally sequenced on the Procise sequencer (Appled Biosystems, Foster City, CA) using the pre-programmed pulsed liquid method for 18 cycles. Apelin-36 and PEG-apelin-36 were also analyzed by MALDI-TOF mass spectrometer (Voyager-DE PRO) (Applied Biosystems, Foster City, CA) in linear mode using DHB (2,5-dihydroxybenzoic acid) as the matrix. The acceleration voltage was 25,000V.

*APJ receptor binding essay*: The binding was performed using competition with ¹²⁵I-labelled [Glp ⁶⁵, Nle ⁷⁵, Tyr ⁷⁷]-apelin13 probe for binding to the APJ receptor in a membrane fraction from the transfected CHO cells (Perkin Elmer, Waltham, MA). Serially diluted peptide was incubated with membrane and probe for 1 hour at room temperature. The mixture was then filtered over a 1.2 um glass fiber type C filterplate (Millipore Corp., Billerica, MA), preblocked with 0.5% polyethylenimine. The plate was washed 4 times with 200ul ice cold binding buffer and then another 4 times with ice cold 50mM Tris-HCl, pH7.4, dried and counted with scintillation fluid. The data was analyzed with Graphpad Prism 4 (GraphPad Software, Inc, La Jolla, CA) using nonlinear regression.

### In vitro bioactivity assay

*Generation of a cell clone with high level APJ receptor expression*: HEK293 cells (ATCC, Manassas, VA) were maintained at 37°C / 5% CO₂ in Dulbecco's-modified Eagle's Medium (DMEM) supplemented with L-Glutamine, penicillin/streptomycin, and 10% fetal bovine serum (FBS). Plasmid pCMV6-XL4 (Origene Technologies, Inc., Rockville, MD) containing a cDNA encoding human APJ was subcloned into pCMV6-Neo (Origene Technologies, Inc.) to generate pCMV6-APJ. HEK293 cells were transiently transfected with pCMV6-APJ using Superfect (Qiagen Technologies Inc., Valencia, CA), and selected in G418 (1 mg/mL) selection media. Clonal cell lines were isolated using sterile filter discs. Surface expression of APJ was detected by flow cytometry using the monoclonal APJ antibody (MAB856, R&D Systems, Minneapolis, MN). A clone expressing a high level of APJ on the cell surface (HEK293-APJ#3) was used to determine the bio-activity of apelin peptides.

*Intracellular cAMP measurement:* HEK293-APJ#3 cells were used to assess the activity of apelin peptides and pegylated apelin derivative peptides. The HEK293-APJ#3 cells were seeded into 96 well plates at 50,000 cells per well. The following day, the cells were pre-treated at 37°C for 5 minutes with assay buffer (0.5mM IBMX in DMEM containing penicillin/streptomycin and 0.5% (w/v) BSA) before addition of apelin peptides (0-300nM) and forskolin (30)µM) prepared in assay buffer. After 15 minute of incubation at 37°C, the apelin / forskolin stimulation was terminated by aspiration of the assay components and addition of Lysis Buffer 1B from cAMP Enzyme Immunoassay (EIA) kit (GE Healthcare, Piscataway, NJ). Lysates (5-50 µL) were assayed for cAMP according to the instructions of the EIA kit. Intracellular cAMP data (apelin-stimulated inhibition of forskolin-mediated cAMP production) was plotted using GraphPad Prism 4 (GraphPad Software, Inc., La Jolla, CA) and fit to a sigmoidal dose-response curve with variable slope.

### In vivo evaluation

*Effect on heart junction in formal rats*: Female Lewis rats, weighing 225-250g (Charles River Laboratories, Wilmington, MA) were anesthetized with Isoflurane (2.5%), driven by oxygen (2.5 L/min) through a nose cone. The body temperature was maintained at 36°C with a water-bath heating pad and complimented with a heating lamp controlled by a rectal probe connected to a TCAT-20F temperature controller (Physitemp Instruments, Inc., Clifton, NJ). The tail vein was cannulated with a 24G Surflo IV catheter. Animals were then randomly divided into 5 groups (n=7/grp) which received either 300nM apelin-36 (Ap36Hi), 30nM apelin-36 (Ap36Lo), 300nM PEG-apelin-36 (Peg36Hi), 30nM PEG-apelin-36 (Peg36Lo), or normal saline (NS) intravenously at a dose of 1ml/20min/25 for 20 minutes. Before infusion, and at 10, 20 (end of IV infusion), 30, 40, 50, 60, and 120 minutes post the initiation of the IV infusion, long axis 2D view echocardiography images were obtained using a 15MHz probe and GE Vivid 7 Dimension echocardiography unit (GE Healthcare Technologies, Waukesha, WI). Anesthesia was stopped between the 60 and 120 minutes time points.

*Effect on heart junction in myocardial infarction (MI) rats*: MI was created by permanent ligation of left anterior descending artery (LAD) in male 250-275g Lewis rats (Charles River Laboratories, Wilmington, MA). Briefly, the animal was anesthetized by IP injection of a cocktail of Ketamine (40-80mg/kg), Xylazine (5-10mg/kg), and diazepam (2-4mg/kg) and body temperature maintained as described in section [0080]. An 18G catheter which was inserted into the trachea and the animal was ventilated using a rodent ventilator ( Model 683, Harvard Apparatus, Holliston, MA). The heart was exposed through a left thoracotomy incision at the 4^{th} or 5^{th} intercostal space. The LAD was ligated using a 6-0 silk at a level 1 to 2 mm distal to the edge of the left auricle. The chest was closed in layers, and the animal allowed to recover in a temperature controlled incubator. Fifty two weeks after the MI, the animals were randomly assigned to receive 30nM apelin-36 (Ap36MI; n=5), 30nM PEG-apelin-36 (Peg36MI; n=5), or normal saline (NSMI; n=5) by IV infusion. Anesthesia, temperature control, and the experiment protocol were the same as described in the section 2.3.1 except that higher levels of isoflurane (3% or to effect) and oxygen flow (3L/min) were employed since the body weight of the animals were between 600 and 650g.

*Effect on blood pressure in normal and myocardial infarction (MI) rats*: Normal male adult Sprague Dawley rats weighing 360±14 g (Charles River Laboratories, Wilmington, MA) were anesthetized and body temperature maintained as described in paragraph [0088]. The left carotid artery and jugular vein were surgically exposed. The left carotid artery was cannulated with a Millar mikro-tip catheter (Millar Instruments, Houston, TX) for blood pressure monitoring and the left jugular vein was cannulated with PE-50 tubing for IV injection. The tip of the Millar catheter was placed into the descending aorta and the end of the catheter was connected to a computer with a Powerlab data requisition system (ADInstruments, Inc., Colorado Springs, CO) to monitor blood pressure. Animals were then randomly assigned to 2 groups (n=5/group) to receive either PEG-apelin-36 or apelin-36 (300nM solution, 0.4ml/kg, IV bolus). In all animals NS (0.4ml/kg, IV bolus) was first injected, followed by IV injection of the peptides. For NS injections, blood pressure was monitored before (baseline), and at 10, 20, 40, 60, 180 and 300 seconds following the NS injection. At the end of this period blood pressure returned to baseline and peptide was then injected. Blood pressure was monitored before (baseline), at 10, 20, 40, 60, 180 and 300 seconds, and at 10 and 20 minutes following the peptide injection. The blood pressure data was analyzed using Chart 5 Pro software (ADInstruments, Inc., Colorado Springs, CO). For blood pressure assessment in MI rats, myocardial infarction was performed, as described in paragraph [0079], in 9 male adult Lewis rats (329±16 g; Charles River Laboratories, Wilmington, MA). Four weeks later, blood pressure effects of apelin-36 was evaluated. The protocol for blood pressure evaluation was as described above for normal animals.

### Apelin blood leves

*Administration of the peptides and blood collection*: Normal female Lewis rats, weighing 225-250g (Charles River Laboratories, Wilmington, MA) were anesthetized, with body temperature monitored and tail vein cannulated as described in the section 2.3.1. In addition, the left carotid artery was cannulated with PE-50 tubing. The animals were then randomly assigned to receive either 30nM apelin-36 (n=5) or 30nM PEG-apelin-36 (n=5) intravenously at a dose of 1ml/20min/25 for 20 minutes. Before, during (10 and 20 min) and 40 min post initiation of the IV infusion, 600 µl of whole blood was collected via the carotid artery cannula using an EDTA coated tube with aprotinin (0.6TIU/ml of blood; Phoenix Pharmaceutical Inc., Burlingame, CA) added. After the blood collection at the 40 minute time point, the PE-50 tube was withdrawn, the carotid artery ligated, and the incision closed. The animal was returned to its cage to recover from anesthesia. The 120 minute blood collection was performed by direct heart puncture after the animal was euthanized with 1ml of Euthasol (Virbac AH, Inc., Fort Worth, TX) IP and the chest opened. The blood samples were centrifuged at 1,600 x g for 15 minutes at 4°C. The plasma was then collected and kept in -80°C for future analysis.

*Plasma apelin 36 concentration*: Plasma apelin-36 concentration was analyzed using an apelin-36 enzyme immunoassay (EIA) kit (Phoenix Pharmaceuticals, Inc., Burlingame, CA) according to the manufacturer's protocol.

### EXAMPLE 3

### Results:

### PEGylation of Apelin

*30kDa and 40KDa PEG-apelin-36 conjugates were successfully produced:* PEG-apelin-36 conjugates were generated by reacting the peptide with PEG aldehydes at acidic pH to favor N-terminal over lysine conjugation. For 30kDa and 40kDa PEG-apelin-36 conjugates, almost all the products were mono-PEGylated species. Significantly more di-PEGylated products were detected in the 10kDa PEGylation reaction. The reaction mix was dialyzed to remove the trace amounts of unreacted peptides. Recovery of mono-PEGylated 30kDa and 40kDa conjugates were >75%. However, recovery of the mono-PEGylated 10kDa conjugate was much lower (<20%) presumably due to its smaller size. Unconjugated PEG was removed by weak cation exchange chromatography with >80% recovery of the PEGylated peptide (Fig. 1).

*PEGylation of apelin 36 occurred at the N-terminal and the purity was* > 98%: N-terminal sequencing suggests that PEGylation of apelin-36 was highly selective for the N-terminus. When the same amounts of PEGylated and unmodified peptide were sequenced, 2 pmol of the first residue of apelin-36 was detected for the PEGylated peptide compared to 146 pmol of the unmodified peptide, indicating ∼98% selectivity for the N-terminus (Fig. 2). To assess the level of unreacted peptide contaminant, the final material was run on a 12% Bis-Tris Gel with free peptide. With 8ug of conjugate loaded, no free peptide was observed, suggesting that the level of free peptide impurity was <2%.

*N-terminal PEGylation of apelin-36 had minimal impact on binding*: The 40kDa PEG conjugates retained high affinity binding with a Kᵢ of 0.3 nM compared to a Ki of 0.05 nM for apelin-36 (Fig. 3). In contrast, di-PEGylated 10kDa PEG apelin-36 conjugate exhibited a Kᵢ of ∼1 nM. The 13 amino acid α-MSH peptide negative control showed no binding.

### In vitro bioactivity (cAMP) assay

A clonal cell line (HEK293-APJ#3) expressing a high level of the APJ on the cell surface was identified by flow cytometric analysis. When challenged with apelin-36, this clone demonstrated suppression of forskolin-mediated cAMP production with an IC50 of 1nM. We next sought to determine whether addition of the PEG moiety to apelin-36 altered its properties as a peptide agonist of APJ. As shown in Figure 4, the IC50 of the PEG-apelin-36 was modestly shifted to the right to 1.5nM, with no change in the maximal inhibition of forskolin-mediated cAMP accumulation versus apelin-36. These results indicate that the PEG moiety does not interfere with the agonist properties of apelin-36.

### In vivo evaluation

*PEG*-*apelin 36 demonstrated prolonged inotropic effect in normal rats*: Rats received a 20 minute infusion of a low dose of apelin-36 or PEG-apelin-36, a high dose of apelin-36 or PEG-apelin-36, or saline and cardiac function was monitored by echocardiography at specified time points during the infusion and for 100 minutes after the end of infusion. PEG-apelin-36 demonstrated prolonged inotropic effects in normal rats. During infusion, apelin-36 and PEG-apelin-36 groups demonstrated similar significant increases in EF with peak EFs observed at the 20m time point. After the infusion ended the decline in EF was slower in the PEG-apelin-36 groups compared to the apelin-36 groups. By 30 minutes after the infusion ended (50 min time point), the EF in the apelin-36 low dose group (Ap36Lo) was not significantly different from the baseline (BL) or the normal saline (NS) group. In the PEG-apelin-36 low dose group (Peg36Lo) EFs were significantly increased compared to baseline and NS group values for the duration of the experiment (Fig. 5a). Similar changes in EF were observed in the high dose groups (Fig. 5b). Peak EFs for the high dose apelin-36 (Ap36Hi) or PEG-apelin-36 groups (PegAp36 HI) were observed at the end of IV infusion (20 min). In the PegAp36 Hi group, EFs remain above BL and the NS group for the duration of the experiment. In the Ap36 Hi group EF was not significantly different from baseline at 40 min after the end of IV infusion (60 min time point) and was not significantly different from the NS group at 100 min after the end of IV infusion (120 min time point)

*PEG-apelin 36 demonstrated prolonged and enhanced inotropic effect in MI rats*: As observed in normal rats, PEG-apelin-36 demonstrated prolonged inotropic effects in MI rats compared to apelin-36 (Fig. 6). In the PEG-apelin-36 group (Peg36MI) EF values were significantly increased compared to the baseline and the NS group (NSMI) for the duration of the experiment. However, in the apelin-36 group (Ap36MI) EF was not significantly different from BL at 60 min (40 min after IV infusion ended) and was not significantly different from the NS group at 120 min (100 min after the end of IV infusion).

There were no significant changes in mean aortic blood pressure (ABPmean) in normal rats following either PEG-apelin-36 or apelin-36 administration compared to baseline values, nor were there changes in ABPmean in MI rats following apelin-36 administration (Fig. 7).

### Apelin blood levels

When animals received apelin-36, the plasma level of apelin-36 was significantly increased compared to baseline at the 20 min (end of IV infusion) and 40 min (20 min after the end of IV infusion) time points (2.5 and 2 fold, respectively). When animals received PEG-apelin-36, the plasma level of apelin-36 was significantly increased at 10 min (during IV infusion), 20 min (end of IV infusion), 40 min, and 120 min (end of monitoring) time points (2.5, 4, 2.7, and 2,7 fold, respectively) (Fig. 8). The kinetics of the changes in apelin-36 blood levels in animals that received either apelin-36 or PEG-apelin-36 were consistent with the observed changes in cardiac ejection fraction (EF) in these animals.

N-terminal PEGylation of apelin-36, had little effect on APJ binding where the mono 40kDa PEG conjugate had a Kᵢ of 0.3 nM (vs Ki of 0.05 for apelin-36). The di-PEGylated 10kDa PEG apelin-36 conjugate had a Ki of ∼1 nM. This 20-fold loss in binding affinity was likely due to PEGylation at one of the two internal lysines. Severe binding affinity loss observed with N-terminal PEGylation of apelin-12 and apelin-17) indicated that PEGylation at the second lysine in the sequence would probably result in a virtually inactive conjugate. The 30kDa and 40 KDa apelin-36 conjugates had similar Ki's. As the 40kDa PEG apelin-36 was anticipated to have a longer circulating life due to its large molecular mass, this conjugate was selected for further characterization in vitro and in vivo.

The IC50 of the 40 KDa PEG-apelin-36 was shifted only 1.5-fold to the right, with no change in the maximal inhibition of forskolin-mediated cAMP accumulation versus apelin-36. These data indicated that attachment of an N-terminal PEG group to apelin-36 did not compromise its function as an APJ agonist, which was consistent with the receptor binding assay mentioned above and the fact that both apelin-36 and PEG-apelin-36 demonstrated similar magnitudes of EF elevation during the 20 minutes of peptide infusion in our in vivo evaluation.

The short circulating time of unmodified apelin peptides and transient inotropic effects were anticipated. Conjugating apelin-36 with 40Kda PEG, increased the duration of its inotropic effect compared to apelin-36, consistent with the anticipated increase in circulation half-life of PEG-apelin-36. PEG-apelin-36 maintained elevated inotropic effects for at least 100 minutes after the end of the IV infusion. In contrast, with the low dose of unmodified apelin-36 EF returned to baseline and was not significantly different from the NS group at 30 min post IV infusion. In the high dose apelin-36 group EF returned to baseline at 40 min post IV infusion and was not significantly different from the NS group at .100 min after IV infusion.

PEG-apelin-36 IV infusion demonstrated an extended duration of increased EF in MI rats. Interestingly, the magnitude of PEGylated and unmodified apelin-36 mediated inotropic effects was much greater in MI rats than in the normal rats. Peak EF values (20 min time point for all of groups) in MI rats were 30% for the apelin-36 group (Ap36MI) and 40% for PEG-apelin-36 group (Peg36MI). EF values in normal rats that received the same dose of apelin-36 (Ap36Lo) or PEG-apelin-36 (Peg36Lo) were 18% and 21%, respectively.

To elucidate the relationship between increased heart function and apelin-36 blood levels, an apelin-36 EIA was used to measure apelin-36 blood levels in animals that received either apelin-36 or PEG-apelin-36. It was noted that this assay underreported apelin-36 levels in animals that received PEG-apelin-36 possibly due to reduced binding affinity of the anti-apelin antibody to the PEG-apelin-36. Due to this issue, apelin-36 levels in this study were reported as fold change compared to baseline. As anticipated, we found that the extended duration of inotropic effects with PEG-apelin-36 corresponded with an extended duration of apelin-36 blood levels above baseline.

In summary, N-terminal PEGylated apelin-36 was successfully produced with high receptor binding affinity and agonist activity retained. The prolonged inotropic effects and extended circulation time of PEG-apelin-36 were demonstrated.

It will be appreciated by those skilled in the art that changes could be made to the embodiments described above without departing from the broad inventive concept thereof. It is understood, therefore, that this invention is as defined by the appended claims.

## Claims

1. A pegylated Apelin 36 molecule comprising one or more polyethylene glycol (PEG) molecules operably linked to at least one amino acid residue in the N-terminal of Apelin 36.

2. The molecule of claim 1, wherein said pegylated Apelin 36 is capable of exhibiting a prolonged circulating life or an extended inotropic effect, relative to a non-pegylated Apelin 36.

3. The molecule of claim 1, wherein said pegylated Apelin 36 is a mono-pegylated or a di-pegylated Apelin 36.

4. The molecule of claim 1, wherein said one or more PEG molecules are covalently attached to said at least one amino acid residue, either directly or via linker.

5. The molecule of claim 1, wherein each PEG molecule has a molecular weight ranging from about 5,000 to about 50,000 daltons.

6. The molecule of claim 1, wherein said pegylated Apelin 36 comprises monomeric Apelin 36.

7. The molecule of claim 1, wherein said pegylated Apelin 36 comprises an oligomeric Apelin 36, wherein one or more monomers are operably linked to each other.

8. The molecule of claim 1, wherein Apelin 36 comprises the amino acid sequence of amino acid residues 42-77 of SEQ ID NO: 1.

9. The molecule of claim 8, wherein said amino acid sequence is encoded by a nucleic acid sequence 123-234 bp of SEQ ID NO: 2.

10. A method for producing the molecule of claim 1, comprising the step of:
reacting Apelin 36 with an activated PEG-aldehyde linker in the presence of a reducing agent to form said pegylated Apelin 36 under conditions in which the linker is covalently attached to Leucine residue in the N-terminal of Apelin 36.

11. The method of claim 10, wherein the reducing agent is cyanoborohydride and the reacting step is performed at a pH of 5.0 to 7.5.

12. A pharmaceutical composition comprising a therapeutically effective amount of a pegylated Apelin 36 that comprises one or more polyethylene glycol (PEG) molecules operably linked to at least one amino acid residue in the N-terminal of Apelin 36, wherein said at least one amino acid residue is Leucine.

13. A therapeutically effective amount of the pegylated Apelin 36 molecule of claim 1 for use in treating a disease or disorder associated with Apelin, wherein said disease or disorder is a cardiovascular disease, an ischemia-reperfusion injury, myocardial infarction, acute decompensated heart failure, chronic heart failure, cardiomyopathy, endocrine disorder, metabolic disorder or pulmonary hypertension.

14. The pegylated Apelin 36 molecule for use according to claim 13, wherein said at least one amino acid residue in the N-terminal of Apelin 36 is Leucine.

## Patentansprüche

1. Pegyliertes Apelin 36-Molekül, umfassend ein oder mehrere Polyethylenglykol (PEG)-Moleküle, in Wirkbeziehung verbunden mit mindestens einem Aminosäurerest am N-Terminus von Apelin 36.

2. Molekül nach Anspruch 1, wobei das pegylierte Apelin 36 eine verlängerte Zirkulationsdauer oder eine erweiterte inotrope Wirkung in Bezug auf ein nicht-pegyliertes Apelin 36 zeigen kann.

3. Molekül nach Anspruch 1, wobei das pegylierte Apelin 36 ein mono-pegyliertes oder eine di-pegyliertes Apelin 36 ist.

4. Molekül nach Anspruch 1, wobei das eine oder die mehreren PEG-Moleküle kovalent an den mindestens einen Aminosäurerest entweder direkt oder über einen Linker gebunden sind.

5. Molekül nach Anspruch 1, wobei jedes PEG-Molekül ein Molekulargewicht im Bereich von etwa 5.000 bis etwa 50.000 Dalton aufweist.

6. Molekül nach Anspruch 1, wobei das pegylierte Apelin 36 monomeres Apelin 36 umfasst.

7. Molekül nach Anspruch 1, wobei das pegylierte Apelin 36 ein oligomeres Apelin 36 umfasst, wobei ein oder mehrere Monomere in Wirkbeziehung miteinander verbunden sind.

8. Molekül nach Anspruch 1, wobei Apelin die Aminosäuresequenz der Aminosäurereste 42-77 von SEQ ID NO: 1 umfasst.

9. Molekül nach Anspruch 8, wobei für die Aminosäuresequenz von einer Nukleinsäuresequenz von 123-234 bp der SEQ ID NO: 2 codiert wird.

10. Verfahren zur Herstellung des Moleküls von Anspruch 1, umfassend den Schritt des:
Umsetzens von Apelin 36 mit einem aktivierten PEG-Aldehyd-Linker in Gegenwart eines Reduktionsmittels unter Bildung des pegylierten Apelin 36 unter Bedingungen, bei denen der Linker kovalent an einen Leucin-Rest am N-Terminus von Apelin 36 angebracht wird.

11. Verfahren nach Anspruch 10, wobei das Reduktionsmittel Cyanoborhydrid ist und der Reaktionsschritt bei einem pH von 5,0 bis 7,5 durchgeführt wird.

12. Pharmazeutische Zusammensetzung, umfassend eine therapeutisch wirksame Menge eines pegylierten Apelin 36, das ein oder mehrere Polyethylenglykol (PEG)-Moleküle umfasst, die in Wirkbeziehung mit mindestens einem Aminosäurerest am N-Terminus von Apelin 36 verbunden sind, wobei der mindestens eine Aminosäurerest Leucin ist.

13. Therapeutisch wirksame Menge des pegylierten Apelin 36-Moleküls von Anspruch 1 zur Verwendung bei der Behandlung einer Krankheit oder Störung, die im Zusammenhang mit Apelin steht, wobei die Krankheit oder Störung eine kardiovaskuläre Krankheit, eine Ischämie-Reperfusionsverletzung, Myokardinfarkt, akut dekompensierte Herzinsuffizienz, chronische Herzinsuffizienz, Kardiomyopathie, endokrine Erkrankung, Stoffwechselstörung oder Lungenhochdruck ist.

14. Pegyliertes Apelin 36-Molekül zur Verwendung nach Anspruch 13, wobei der mindestens eine Aminosäurerest am N-Terminus von Apelin 36 Leucin ist.

## Revendications

1. Molécule d'apeline 36 pégylée comprenant une ou plusieurs molécules de polyéthylène glycol (PEG) liées de manière fonctionnelle à au moins un résidu d'acide aminé N-terminal de l'apeline 36.

2. Molécule selon la revendication 1, dans laquelle ladite apeline 36 pégylée est capable de présenter une durée de vie prolongée en circulation ou un effet inotrope allongé, par rapport à une apeline 36 non pégylée.

3. Molécule selon la revendication 1, dans laquelle ladite apeline 36 pégylée est une apeline 36 mono-pégylée ou di-pégylée.

4. Molécule selon la revendication 1, dans laquelle ladite ou lesdites molécules de PEG sont fixées par covalence audit au moins un résidu d'acide aminé, soit directement soit par l'intermédiaire d'un lieur.

5. Molécule selon la revendication 1, dans laquelle chaque molécule de PEG possède une masse moléculaire allant d'environ 5000 à environ 50 000 daltons.

6. Molécule selon la revendication 1, dans laquelle ladite apeline 36 pégylée comprend de l'apeline 36 monomère.

7. Molécule selon la revendication 1, dans laquelle ladite apeline 36 pégylée comprend de l'apeline 36 oligomère, dans laquelle un ou plusieurs monomères sont liés de manière fonctionnelle l'un à l'autre.

8. Molécule selon la revendication 1, dans laquelle l'apeline 36 comprend la séquence d'acides aminés des résidus d'acides aminés 42 à 77 de SEQ ID No. : 1.

9. Molécule selon la revendication 8, dans laquelle ladite séquence d'acides aminés est codée par une séquence d'acides nucléiques 123-234 bp de SEQ ID No. : 2.

10. Procédé de production de la molécule selon la revendication 1, comprenant l'étape consistant à : faire réagir de l'apeline 36 avec un lieur activé PEG-aldéhyde en présence d'un agent réducteur pour former ladite apeline 36 pégylée dans des conditions dans lesquelles le lieur est fixé par covalence à un résidu leucine N-terminal de l'apeline 36.

11. Procédé selon la revendication 10, dans lequel l'agent réducteur est du cyanoborohydrure et l'étape de réaction est effectuée à un pH de 5,0 à 7,5.

12. Composition pharmaceutique comprenant une quantité efficace sur le plan thérapeutique d'une apeline 36 pégylée qui comprend une ou plusieurs molécules de polyéthylène glycol (PEG) liées de manière fonctionnelle à au moins un résidu d'acide aminé N-terminal de l'apeline 36, dans laquelle ledit au moins un résidu d'acide aminé est la leucine.

13. Quantité efficace sur le plan thérapeutique de la molécule d'apeline 36 pégylée selon la revendication 1, utilisable pour le traitement d'une maladie ou d'un trouble associé à l'apeline, dans laquelle ladite maladie ou ledit trouble est une maladie cardiovasculaire, une lésion d'ischémie-reperfusion, un infarctus du myocarde, une insuffisance cardiaque décompensée aiguë, une insuffisance cardiaque chronique, une cardiomyopathie, un trouble endocrinien, un trouble métabolique ou une hypertension pulmonaire.

14. Molécule d'apeline 36 pégylée utilisable selon la revendication 13, dans laquelle ledit au moins un résidu d'acide aminé N-terminal de l'apeline 36 est la leucine.


### SEQUENCE LISTING

<110> Genzyme Corporation
<120> PEGYLATED APELIN AND USES THEREOF
<130> P-75918-EP
<140> EP 12757055.4
   <141> 2012-03-08
<150> PCT/US12/999999
   <151> 2012-03-08
<150> 61/451,623
   <151> 2011-03-11
<160> 2
<170> PatentIn version 3.5
<210> 1
   <211> 77
   <212> PRT
   <213> Homo sapiens
<400> 1
   Met Asn Leu Arg Leu Cys Val Gln Ala Leu Leu Leu Leu Trp Leu Ser
   1               5                   10                  15
   Leu Thr Ala Val Cys Gly Gly Ser Leu Met Pro Leu Pro Asp Gly Asn
               20                  25                  30
   Gly Leu Glu Asp Gly Asn Val Arg His Leu Val Gln Pro Arg Gly Ser
           35                  40                  45
   Arg Asn Gly Pro Gly Pro Trp Gln Gly Gly Arg Arg Lys Phe Arg Arg
       50                  55                  60
   Gln Arg Pro Arg Leu Ser His Lys Gly Pro Met Pro Phe
   65                  70                  75
<210> 2
   <211> 8271
   <212> DNA
   <213> Homo sapiens
<400> 2
   agcggcgggg ctcagcgcgc acactcagcg gccggggagc ctcccgagct ctgcgcccgc      60
   acgcgccagc cgcggctcgc gcctttcttg gcctccgggc gcccgacctc tcctcccccg     120
   cgccggctcg ccggggccgc ggcggcccaa ggagcagcat gaatctgcgg ctctgcgtgc     180
   aggcgctcct gctgctctgg ctctccttga ccgcggtgtg tggaggtgag tgcgcgccct     240
   cccgctcgcc cgggctgcag ccggcgtgct cctttccctc cgccctattc gcactctccc     300
   cgcctcccgg ggagcccggc gcgagccgtg cagtagcagc cacgcgccgg ccagcgccag 360
   gcagcagatt gacagagcgc cccgagccat ggggcggctg gccacaagga ctgcttcttt 420
   tgcggggctc aactcggcct ttggcatcac ctgcccgtca ccttgtccct cctaaccgtc 480
   gctcgcagtc ccaggagctc aggaccacag gtgtgcgccg cgtgtcccac gccctgcacc 540
   ccgcgtggta gaggtggagg cgggggacat gcccgctccc tcgtggatcg atggaaggaa 600
   ctgaccagag tcgagtgggc tgggctccca tgttgaacgt cttctggtcc ctgaaggctg 660
   ggagcctccc gccagtccag gggcgcggga aaccggggat gatgtgccaa catgcagacg 720
   tgacacttgt tgcataggca ggagtgcgcg gagggggtgg cggcatggag cccagctgtt 780
   gctcctttcg gcctgcatgt gtgagcagag gcagcctccc ccaggcaggg ccgggcccag 840
   ccgggctggg ctggtcttct cctccctaac cccaaggtcc tggctggtct cctcccctgc 900
   acaggatgcc ctccccatag cctccatccc acccccagag acttggttct cttacatcct 960
   ggctccagcc ttcctttctg tcccaggcac ggacatgctt cactttagtc tccctgccgc 1020
   taggcaccaa aggaggaagc acagcacctg acccgacccc tccctgcagc tcaggaaagg 1080
   ggagtccttt tgccctcacc tgccaatact tggcactagt gcccatgtcc gcccctagcc 1140
   tggtccccaa ccacgcccca gcagccccat cccaggctgc ctgtcctctg tgtcaatgtc 1200
   tcctggggct gccagctccc acctccagct gccacagtcc tatgatctgg ctgtcattgc 1260
   tgcctgcctg gcaccacaca aggggaaggc caagaccagg tggactaacg ccaggaccat 1320
   ggctggagtc acagggaggt gggagcagaa agaaggagaa ggcagggcag tgagtttcct 1380
   ggggcaaggg cacagggccc taagtggttc caaaccctcc agaacaggag gatggcatgc 1440
   agcccaggag cctccgaggg gcccagccag ctctgcctgc ctctcatcgc aggccacctc 1500
   ctttctctgc tcttgctttt tggccctgct gtggctctgg gaacactgcc tggcttctca 1560
   gaaaggtgca gtggttgggg ctggcacctc ctgcctccag gtagctggcc ggtgatttct 1620
   ctattcaaga cctgaacacg aagtgagcca ccaccacagt aagaagtggg taggcttttt 1680
   ccacagaaac tcctgtcagg caggtaacag agggagcaaa agatacgaat gtaagatacg 1740
   aatgatacac cataccagcc tatcctcagg gagcctacca tctggtgtca ttggcaaggc 1800
   atgacacttg cacaatacca ccacaggcaa atagtccagg gtggtgcctt cagggaagaa 1860
   gaagactggg tgcagagagt gttccagagg aaggtagggc tcctggatct gcacacttgg 1920
   tgctcccccc agaggctgtg gggagacccc tggccaatag acaaccactc agaccctcct 1980
   gggtcccagc cattgggaca gtactagcaa aggggagaga gctggtttca agggaaggga 2040
   agtcagccag gatgcccgaa caccatgtgg ctggtccaga gcctgccact tcgtgcttta 2100
   attcccccgt cttcattagg tcatctgtcc actgtgattt ggataaattc gttgccctca 2160
   cattttgctc aagcctcgtg ctggaaactt tcagggctct cactgacttc tctactccct 2220
   gacttctcct ggagccaagg aagggcatgt ccctggaagt ggccaccagc caagggtcca 2280
   ggtacacatg tcctgctccc gctggctcct gtcctgggtt agaagcagga atcccagaac 2340
   cactttgtcc ttggcccgga ctggcatgct gtccccagcc agagtcccca gtcaaagttc 2400
   cagcctgaaa accacacttc ctcctctcca cagtctgtcc tgacttgaaa tgtccacccc 2460
   cgacccctct ccacgctgcc tctcatcacc ccgctaccac cacgtacact cctccactgc 2520
   ctcctgacct gggatacact ttgctcttgt gagtctgaga actcctggtc cagggccaag 2580
   tgtccctcgg tctctgagca cccctctttt tccccttctc ctccccctcc gcaggcccag 2640
   ctcaaagcct gcagtcacag tgggagtgtg aggagagcct actgtacccc tcctgctcag 2700
   ggaagggcat ctctactcca tccccagtcc tcagcactaa gcacccgtgt aacctgagcc 2760
   aggcctctcc tcgcaaagaa ctaaggcaag ggaagcagag ggccaccaga cagagcatga 2820
   tgtatcccag ggcagcaaag ctggcactgg agacagcaca gggccctgga gaaggcgact 2880
   gggatggggg gctgggcaga caaagagaca catgcaggac cacagagcag tggaggggca 2940
   ttgccagcag ggtccccacc ggagcatacg tttgggcttt taaagtcatc acaagacagg 3000
   ccagactgag agaggaaaaa caggtgcgtc attcctgatg agactggcat gttcctgaaa 3060
   agtcgcaagt acctctttgt gcattggcag ggttggagtt cagatagcat gaaagtcaaa 3120
   tcctgtcctc cccctgactg tggttctcag cgtggagatg ttgtagcttc gtggctgatg 3180
   gcacttcagc aggcaatggc tccaatctct ctaggctcga gattgtttgg aggattgctc 3240
   cagcctcctt ttacgtagca aatgagctgg aagaaaatat ttaagtgcat tagggctggc 3300
   ctccatttct aaaggggaat tcttttttac agtcaagact tctgcagtga tgagaatcaa 3360
   cagcctctaa tttgttcctt tggacagcat aaagccaggg tttcagatgt cagcttatct 3420
   gatggtgagc cagactcaga atcaaaagta ggaaacaggt gggtgacggg ctcagagtgc 3480
   gggaggagcc agcagatctc ggtgatgctg aggacaatga gtccccaacg caatgggagg 3540
   gatgcaagaa gcctggggct gctctccagg gcccggctgg gggttcagtc agctcagagt 3600
   ggttcttctc tgcagaccta gagtctagac atcttcctgg ccagagccac caaccctgct 3660
   ccccatcctg acatatggcc tctagccctc cctttctgac tgccagacac tcacaatctg 3720
   agcacctgga gatgatgccc tccccctcct ggcaccatgc ttctcttctc catgtctgcc 3780
   accaccctga gctacaatgg cctgcaccac tgccccatcc cttctgcacc cccagcagtc 3840
   gaaggtgcag aggtgagagc caagtgttga cttggttcac ttcacaaggc ccctaggtcc 3900
   ttcccctgct ccttatcagc atcattcaga tccgccctca gtgatcaggg tgagccttgt 3960
   ggggacccca gaactcaagg cagaggctga agcagctgca gtggggagct tagtgaaggt 4020
   ctttccttcc tttccctctc cagccccagc cccctcacag atggcgccaa gagggtagct 4080
   ctatgacagt cagacagaag cagccccctt gttccatcca tacttactgc tccaaagtgc 4140
   taagataatt cttcttttaa ttctcatagg ttatgaattt aagggataag ttggcacgga 4200
   agtgtggcta gttaaccaaa tgacccaagt caacagcggg gtcctcatgg gcctggcaat 4260
   ttttcttggg gaaagaggga aggagggtgc aggcttggag aatatgtgtt tatgtgtgtg 4320
   tgtgtggagg gggtgggtat gtctgggtgt gagtggggag aactgcaggc tcagaagcct 4380
   gggggaagag ggccctaagg ccaaacccac ccacttttgg cccaagagga ggctgaacac 4440
   ttggaaattg tgtgcaagca gaggaggact cacaaaaacc aaggagccaa ggaaggaaca 4500
   gagcgtgggg atgttggagg cagaggcaga ggggaagggg gagctgccag gggtatgatt 4560
   ggggtgtgag cagccaatgg gaggctctat ggaaactgtg agcaagtggt gggaattcct 4620
   gggtagatgg caatggggct cgagggacag ggatctagat gcaggcaaat cagatggtgt 4680
   gcagggggag agagactgtt attcattatc tctggcctca gatgctgtat ctgcacaggc 4740
   ctgtactgga cattgcacaa gtaccctgcc tggctgcctc ttccccagca ccatctcatc 4800
   tccacacact gcctgccacc accagccagg cttgtcttgg gggtggcagc tcagcttccc 4860
   tgcatggact gcaggcttca cgagagtcgg cctgcatgcc ttttccctca ctgcacccaa 4920
   ggataccctg caccacttgg attccattgt cagggctcag tctggtgtca ggaggcctct 4980
   actctaagcc tcctgacaga tgtggacaga tgtaaagagg agccccagag tgcagaatag 5040
   aagccagctg ggattgaaat ggaaggaatt tacatgagac acaggatgca gggtgtgatc 5100
   tgggaatttc aggaggtagg gcattggtgg ggaggtacca actgcaacag cccacccccc 5160
   tcctggtgca cagacatgct gctgaaggtg accattgttt catttattca ttctcaaata 5220
   ttcatgcaat atttattggt ctctaaatct ggtctcaggt ctctaaatct ggtcacagca 5280
   tgaggcaggc tgagtcagag acctctcccc cttaacccgc ctggcccagc cccatccact 5340
   gggaggactt ggcgccctag agatgcctaa ccaccgatga gacacccagg ggcgagatat 5400
   gagctcccta gtactcacaa gtgggtcatc ctgcatatca aaggatgtag gagtacaatg 5460
   acgtgatgtt cgggatttgc tttaaaatac tgcagtcaag gaaaatagaa aagggataac 5520
   taaagcatat gtgactccat ctggataact gttgaatctg ggtgatgggt ccatggcggt 5580
   gggagggtca ttgtaatgtc atatgtctga aatgtttgaa gtgtaccagt tgcaggggtg 5640
   ccgtggtgat tactgcctcc tgccccccag ccaagtacag gcgtgacaac ccaaagtgca 5700
   caccccccaa tgacgttcca aagaaagaac ccacttgtgc ctgtcagaaa catgatataa 5760
   ctgtcaaaag gacagaaagt cagctacatc aatggcaaga tgtgagtagt aggttccgtt 5820
   tgtcccagga cagtgcagaa atggcaaatt ttccctcaat atgaaaaaaa gaaacaaaga 5880
   aaatctatga gaagtgccac cacatggaca gaccacccct cccgcgggcc tgtggtctgc 5940
   agcccctgcc cgtttccaac aaccttcctc actaacaggc tttctccttc ctgcagggtc 6000
   cctgatgccg cttcccgatg ggaatgggct ggaagacggc aatgtccgcc acctggtgca 6060
   gcccagaggg tcaaggaatg ggccagggcc ctggcaggga ggtcggagga aattccgccg 6120
   ccagcggccc cgcctctccc ataagggacc catgcctttc tgaagcaggt acttgagcct 6180
   ctgttcagtg gacagtgcta aagcggaggg agaaggacag cctagaagcc gggtgatcct 6240
   gccgctcatc actggccaga cacatccatc aaacagtcct ggcgccacac tcacagaggc 6300
   ttagagctgg caagagagct tatggggcag ctgtctagcc cctttatttt acagacgatc 6360
   tcctcgtctg aggcctagag aggggcttga tacttggggc cagtctccct tccaccctgc 6420
   tgcctggggt tggggaggct ggcctgctta gagatttcta gacatcctgg tggaccttaa 6480
   tggctgggga ggggtgccca gggcaaacct acagatgtcc agtgtagatt gtcgccagag 6540
   ttggggaagg aggcagagag ctctaggggc tctcaggcag agaacaagag gacagttgtg 6600
   gctgtggttg cccggagctg tgagaaggag ggaagaacca aggtggcagc agagagagga 6660
   gagctgaagc ctgccccttc cctggtctgc tggctgtccc actgcagtgg ggacaggcag 6720
   gcaccaactc aggggccttg ggattgaggt ttgggctgag tggagtttgg gggaaagaga 6780
   aggcggtctc ctctctcctc tcctttcctg gggagttcct ttggcctagg atatgtccat 6840
   gacttctctc ttgtcatttt tgtctcctta ggactgaagg ggcccccaag tgcccacccc 6900
   cggcggttat gtctcctcca tagattggtc tgcttctctg gaggcctcac gtccattcag 6960
   ctctcacctc gcacctgctg tagccaccag tgggcccagc tcttctcacc tgcctgcttc 7020
   ccccagtggc gtgctcctgg ctgtagtttg gatgattccc gttctctcac aagaatccgt 7080
   ccagtccatc ttcctggccc ctccctggac tgactttgga gacctagccc cagaaagcct 7140
   cccttcttct ccaggtcccc tccgccctag tccctgcctg tctcatctaa cgccccaaac 7200
   cttcatttgg gccttccttc ctcatgtctg ccctgagcgc ggggtggaag tgctcccttc 7260
   tgtgggctcc agcagatccc ttgttttcct gtcagttgga cccctcacct ggcctccagg 7320
   gaagaatgca gagaaaagca aggagagact ctagttaaga ggtgctggct gcggggatcc 7380
   agacagggca cattgggggc atggaagtgc cagggtggtt ttcaggagct ctggtgaagt 7440
   gggtggagca tcagcgtttg ctcagttaag ggagaggtag agaggggccc gtgaagtcct 7500
   ttgtcacttc tcttgcctta gtgtgcctcc caatactccc ttcttcctgc ccccacaccc 7560
   catccccagc tagcccaagc tccaggtcag gaggggaggg tgctgggcct gacatggcta 7620
   tataccctcc caggagtaaa agccaagcaa gaggttgttt ttgccaagaa tcacagaatg 7680
   ttagagctga caggaccctt gaaggtcact tagccttctt aggcaaacgc ctgcaaaaca 7740
   gaagcctgga gaggggagtg acctgctcag agtcattgca gagccgggat ggggaccagg 7800
   tctcccatct cctactttat gacgccctct tccctcttga tgatgtcttt tcaaagcaaa 7860
   tgaagtgcct tttcccgagg ctggggctgg gggtggctgg gaggggaagg gaagggagag     7920
   gcaagctggc tgtgaactgt cctgttgtgg ggctggagct gctcccacct ccctgaccta     7980
   cccctgctgc accattcccc cagctgggct ggaaggttcc ataactggcc agctgccccc     8040
   ataactggca gcattcccag acccagggta ctctaatagg ggcggctcag gcactgagac     8100
   taccgctcaa ccccagggtg gttttcagga gtccgaggta gccttcaatc actggactcc     8160
   atggccttcc cttcgtgttg accggacctt ccttccaggg cttttccttt gggggaggcg     8220
   gagaggggag aagaaggaag ggaagggcag aaggaaggag ggaagaaaag a              8271